# EUROPEAN PATENT SPECIFICATION

(11) **EP 4 046 669 B1**
(45) Date of publication and mention of the grant of the patent: **16.04.2025**
(21) Application number: 21209073.2
(22) Date of filing: 18.11.2021
(51) Int. Cl.: A61M 3/02, A61B 1/015

(54) **FLUID MANAGEMENT SYSTEM**
FLÜSSIGKEITSMANAGEMENTSYSTEM
SYSTÈME DE GESTION DE FLUIDES

(30) Priority: 17.02.2021 GB 202102202
(43) Date of publication of application: 24.08.2022
(73) Proprietor: KEYMED (Medical & Industrial Equipment) Limited, Southend-on-Sea Essex SS2 5QH (GB)
(72) Inventor: TRIGG, Daniel, SOUTHEND-ON-SEA, SS2 5QH (GB)
(74) Representative: Boult Wade Tennant LLP

(56) References cited:
- EP-B1- 0 929 263
- WO-A1-96/13216
- US-A1- 2020 405 955

## Description

The present invention relates to a fluid management system for medical procedures such as endoscopies.

Endoscopic procedures involve the insertion of a medical instrument into a patient's body to observe an internal organ or tissue in detail for the purposes of imaging, investigation, diagnosis or surgery/treatment. Such endoscopic procedures often involve supplying irrigation fluid into the patient's body to irrigate and clean the site being observed. For example, in gastrointestinal (GI) procedures irrigation is helpful to wash gastric and colonic mucosa remove blood, faeces or other organic matter. Irrigation fluid is supplied by a pump into a channel in the endoscope itself. The surgeon carrying out the procedure may operate the pump by means of a footswitch.

US 2020/405955 describes a fluid management system including a pump to supply fluid to a medical device, a footswitch to actuate the pump, a central processing unit to control the pump and a user input device to allow a user to set parameters for pump operation.

EP 0929263 describes a fluid management system in which the user interface is disabled when a footswitch is engaged.

The present invention provides a fluid management system for an endoscopic procedure, the system comprising a pump configured to deliver liquid from a reservoir to a medical device, the pump comprising a central processing unit (CPU) configured to control operation of the pump, a footswitch in communication with the CPU configured to actuate the pump, and a user input device configured to program the CPU to operate the pump at a plurality of predetermined levels of an operational parameter of the pump and to allow a user to select one of the plurality of predetermined levels for pump operation, characterised in that the CPU is further configured to disable at least parts of the user input device when the footswitch is engaged the user input device comprises increment controls operable to incrementally increase and decrease an operational parameter of the pump, and preset controls operable to switch directly between the plurality of predetermined levels of the operational parameter of the pump, and the CPU is configured to disable the preset controls when the footswitch is engaged and to maintain the increment controls in an enabled condition when the footswitch is engaged.

The improved fluid management system of the present invention prevents sudden changes in an operational parameter of the pump during use, such as the flow rate of irrigation fluid, in order to prevent potential harm to the patient or damage to the equipment.

The user input device preferably comprises a plurality of buttons on the pump body which are illuminated when enabled.

In a preferred embodiment, the pump comprises a peristaltic pump and the operational parameter is at least one of pump speed or flowrate.

The present invention will now be described in detail, by way of example only, with reference to the accompanying drawing in which:
Figure 1 is schematic view of a fluid management system in accordance with the present invention; and
Figure 2 is an enlarged view of the user input device.

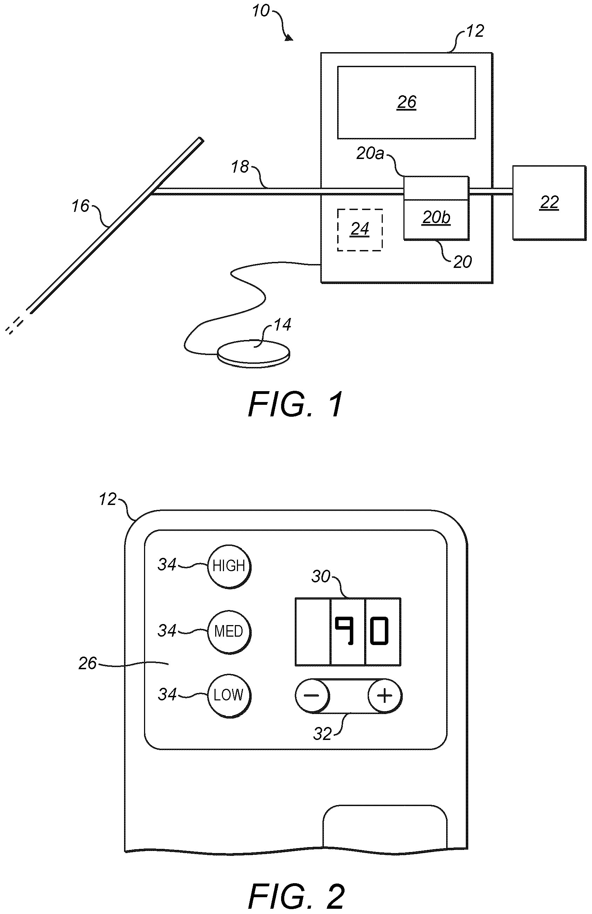

As shown schematically in Figure 1, a fluid management system 10 in accordance with the present invention comprises a pump 12 and a footswitch 14 connected to the pump 12. When the footswitch 14 is engaged, the pump 12 supplies a medical instrument 16, such as an endoscope, with fluid via tubing 18.

Preferably, the pump 12 is a peristaltic pump which houses a drive or motor (not shown) for operating a pump head 20. The pump head 20 includes a track 20a and rotor 20b. Flexible tubing 18 is fitted between the track 20a and rotor 20b and in operation fluid is withdrawn from a reservoir 22 and pumped through the tubing 18. The pump 12 also includes a central processing unit (CPU) 24, which controls operation of the pump 12. A user input device 26 is also provided. The user input device 26 is preferably in form of a control panel on the body of the pump 12, although it could also be a remote device in wired or wireless communication with the pump 12. The CPU 24 is programmable by the user input device 26 to alter certain parameters of operation of the pump 12.

The user input device 26 preferably includes a number of controls operable by a user to change one or more parameters of pump operation, and a display 30, as shown in Figure 2. The adjustable parameters of pump operation may be, for example, the motor speed, the rotations per minute (RPM) of the rotor 20b, or the flow rate of fluid in the tubing 18. The display 30 may indicate an actual value of an operational parameter, or a percentage value. For example, in Figure 2 the display 30 shows a value of 90 which may represent 90% of the maximum available flow rate which the pump 12 can provide.

The footswitch 14 is connected to the pump 12 and in communication with the CPU 24. When the footswitch 14 is pressed by a user the CPU 24 will cause the pump 12 to operate. When the footswitch 14 is disengaged, the CPU 24 ceases operation of the pump 12.

The controls of the user input device 26 preferably include increment controls 32 which allow a given parameter to be increased or decreased in successive small increments. The increment controls 32 may consist of two buttons marked + and - as illustrated, to increase and decrease the parameter, although other forms of control could be used, such as a rotatable knob or wheel, or a slider. The increment controls 32 may be configured to alter a parameter by any desired increment, such as 5% or 10% or some other value.

The controls of the user input device 26 also include preset controls 34 which allow a user to set, and shift between, a plurality of predetermined levels, or presets, of a particular parameter. The pump 12 can be programmed to operate with a given parameter at certain levels, in particular at certain percentages of the maximum available level which can be provided by a given pump. For example, a user may wish to use different fluid flow rates for different procedures or at different times during a procedure. To facilitate these changes and to avoid the need to repeatedly operate the increment controls 32 to increase or decrease the flow rate (or other chosen parameter) prior to a procedure being carried out, presets may be entered corresponding to the desired flow rates. For example, the presets may be a maximum flow rate of 90% of the pump's operational capacity, a medium flow rate of 50% and a minimum flow rate of 10% may be set.

The preset controls 34 preferably comprise three buttons representing maximum, medium and minimum levels. The preset controls 34 may be labelled accordingly, or labelled High, Med and Low, as in Figure 2, or identified in any other convenient manner. More or fewer buttons may be provided depending on the number of presets required, and other forms of control such as switches could be used. The chosen parameter may be set initially by using the increment controls 32. When the desired level is reached, as shown on the display 30, the corresponding preset button may be held for a several seconds to set that level as the desired preset. For example, if the maximum required is 90% as shown in Figure 2, the High button may be held for several seconds to fix that value as the high preset. The High button is then released, the level of the chosen parameter lowered to a required medium value using the increment controls 32, and that level is fixed by holding the Medium button for several seconds and so on. Once the preset controls 34 are all programmed in this way, pressing one of the buttons automatically changes the parameter to the given preset level.

In use, the pump 12 is programmed by a user, using the user input device 26, with the desired parameter levels. These levels may be based on recommended levels for given procedures or may be chosen by an individual user based on their knowledge and experience. During a procedure, the user chooses a desired level by pressing the relevant preset control 34 and then engages the footswitch 14 in order to start and stop pump operation. To change the parameter, e.g. the flow rate, the user presses one of the buttons of the user input device 26. Small changes may be made by using the increment controls 32 and larger changes by choosing a different one of the preset controls 34.

However, if the wrong preset control 34 is pressed inadvertently, e.g. if a change from minimum to maximum is chosen by mistake when a change from minimum to medium was intended, or if the parameter levels which have been programmed as the relevant presets have in fact been altered prior to the procedure being carried out and are not what the user is expecting, there is a risk of a sudden increase or decrease in the parameter, such as flow rate, which could cause injury to the patient, damage to the endoscopic equipment, or otherwise interfere with the efficient carrying out of the procedure.

Therefore, in the present invention the system is configured to prevent any alteration to the presets, or switching between presets, while the pump 12 is in operation. The user input device 26, at least in so far as it relates to the preset controls 34, is disabled while the pump 12 is operating, that is, while the footswitch 14 is engaged. The relevant parts of the user input device 26 are disabled by means of the CPU 24, which is configured to detect engagement and disengagement of the footswitch 14 and is programmed to disable the preset controls 34 when the footswitch 14 is engaged so that step changes from one preset level to another, or changes to level of each preset, are not permitted.

However, the increment controls 32 are not disabled so that the parameter can still be altered during operation of the pump 12, but only small incremental changes are possible. Therefore the parameter, such as the flow rate, can be changed but only gradually in a way which will not cause injury or damage.

The control buttons on the user input device 26 may be illuminated when they are enabled. Preferably, the CPU 24 is also operable to stop illumination of the disabled preset control 34 when the footswitch 14 is engaged, to make the user aware that they are inoperable. Preferably, the increment controls 32 remain illuminated even when the footswitch 14 is engaged, to ensure a user is aware they are still operable and the flow rate changes can be made incrementally.

## Claims

1. A fluid management system (10) for an endoscopic procedure, the system (10) comprising a pump (12) configured to deliver liquid from a reservoir (22) to a medical device (16), the pump (12) comprising a central processing unit (CPU) (24) configured to control operation of the pump (12), a footswitch (14) in communication with the CPU (24) and configured to actuate the pump (12) , and a user input device (26) configured to program the CPU (24) to operate the pump (12) at a plurality of predetermined levels of an operational parameter of the pump (12) and to allow a user to select one of the plurality of predetermined levels for pump operation, **characterised in that** the CPU (24) is further configured to disable at least parts of the user input device (26) when the footswitch (14) is engaged, the user input device (26) comprises increment controls operable to incrementally increase and decrease an operational parameter of the pump (12), and preset controls operable to switch directly between the plurality of predetermined levels of the operational parameter of the pump (12), and the CPU (24) is configured to disable the preset controls when the footswitch (14) is engaged and to maintain the increment controls in an enabled condition when the footswitch (14) is engaged.

2. A fluid management system as claimed in claim 1, wherein the user input device (26) comprises a plurality of control buttons on the pump body which are illuminated when enabled.

3. A fluid management system as claimed in claim 1 or claim 2, wherein the pump (12) comprises a peristaltic pump.

4. A fluid management system (10) as claimed in claim 3, wherein the operational parameter is at least one of pump speed or flow rate of liquid being pumped.

## Patentansprüche

1. Fluidmanagementsystem (10) für ein endoskopisches Verfahren, wobei das System (10) eine Pumpe (12) umfasst, die konfiguriert ist, um Flüssigkeit aus einem Reservoir (22) an ein medizinisches Gerät (16) zu liefern, wobei die Pumpe (12) eine zentrale Verarbeitungseinheit (CPU) (24), die konfiguriert ist, um den Betrieb der Pumpe (12) zu steuern, einen Fußschalter (14), der mit der CPU (24) in Verbindung steht und konfiguriert ist, um die Pumpe (12) zu betätigen, und ein Benutzereingabegerät (26) umfasst, das konfiguriert ist, um die CPU (24) zum Betreiben der Pumpe (12) bei einer Mehrzahl von vorgegebenen Niveaus eines Betriebsparameters der Pumpe (12) zu programmieren und einem Benutzer zu ermöglichen, eines der Mehrzahl von vorgegebenen Niveaus für den Pumpenbetrieb auszuwählen, **dadurch gekennzeichnet, dass** die CPU (24) ferner konfiguriert ist, um zumindest Teile der Benutzereingabevorrichtung (26) zu deaktivieren, wenn der Fußschalter (14) betätigt wird, wobei die Benutzereingabevorrichtung (26) Inkrementsteuerungen, die zum inkrementellen Erhöhen und Verringern eines Betriebsparameters der Pumpe (12) betreibbar sind, und Voreinstellungssteuerungen umfasst, die zum direkten Umschalten zwischen der Mehrzahl vorgegebener Niveaus des Betriebsparameters der Pumpe (12) betreibbar sind, und die CPU (24) konfiguriert ist, um die Voreinstellungssteuerungen zu deaktivieren, wenn der Fußschalter (14) betätigt wird, und um die Inkrementsteuerungen in einem aktivierten Zustand zu halten, wenn der Fußschalter (14) betätigt ist.

2. Fluidmanagementsystem nach Anspruch 1, bei dem die Benutzereingabeeinrichtung (20, 26) eine Mehrzahl von Steuerknöpfen auf dem Pumpenkörper umfasst, die beleuchtet sind, wenn sie aktiviert sind.

3. Fluidmanagementsystem nach Anspruch 1 oder 2, bei dem die Pumpe (12) eine peristaltische Pumpe umfasst.

4. Fluidmanagementsystem (10) nach Anspruch 3, bei dem der Betriebsparameter mindestens einer von Pumpendrehzahl oder Durchflussrate der gepumpten Flüssigkeit ist.

## Revendications

1. Système de gestion de fluide (10) pour une intervention endoscopique, le système (10) comprenant une pompe (12) configurée pour distribuer un liquide depuis un réservoir (22) vers un dispositif médical (16), la pompe (12) comprenant une unité centrale de traitement (CPU) (24) configurée pour commander l'actionnement de la pompe (12), un commutateur à pied (14) en communication avec la CPU (24) et configuré pour actionner la pompe (12), et un dispositif d'entrée utilisateur (26) configuré pour programmer la CPU (24) pour actionner la pompe (12) à une pluralité de niveaux prédéterminés d'un paramètre d'actionnement de la pompe (12) et pour permettre à un utilisateur de sélectionner l'un de la pluralité de niveaux prédéterminés pour l'actionnement de la pompe, **caractérisé en ce que** la CPU (24) est en outre configurée pour désactiver au moins des parties du dispositif d'entrée utilisateur (26) lorsque le commutateur à pied (14) est appuyé, le dispositif d'entrée utilisateur (26) comprend des commandes d'incrément utilisables pour augmenter et diminuer de façon incrémentielle un paramètre d'actionnement de la pompe (12), et des commandes préréglées utilisables pour commuter directement entre la pluralité de niveaux prédéterminés du paramètre d'actionnement de la pompe (12), et la CPU (24) est configurée pour désactiver les commandes préréglées lorsque le commutateur à pied (14) est appuyé et pour maintenir les commandes d'incrément dans un état activé lorsque le commutateur à pied (14) est appuyé.

2. Système de gestion de fluide selon la revendication 1, dans lequel le dispositif d'entrée utilisateur (26) comprend une pluralité de boutons de commande sur le corps de pompe qui sont éclairés lorsqu'ils sont activés.

3. Système de gestion de fluides selon la revendication 1 ou la revendication 2, dans lequel la pompe (12) comprend une pompe péristaltique.

4. Système de gestion de fluide (10) selon la revendication 3, dans lequel le paramètre d'actionnement est au moins l'un d'une vitesse de pompe ou un débit de liquide pompé.
